(19) Europäisches Patentamt
European Patent Office
Office européen des brevets

(11) Veröffentlichungsnummer : **0 104 602**
**B1**

(12) **EUROPÄISCHE PATENTSCHRIFT**

(45) Veröffentlichungstag der Patentschrift : **10.09.86**

(51) Int. Cl.[4] : **C 11 B 9/00**, C 07 C 43/04, C 07 C 43/15, C 07 C 41/20

(21) Anmeldenummer : **83109394.3**

(22) Anmeldetag : **21.09.83**

(54) **Riechstoffkompositionen mit einem Gehalt an Äthern.**

(30) Priorität : **27.09.82 US 423901**

(43) Veröffentlichungstag der Anmeldung : **04.04.84 Patentblatt 84/14**

(45) Bekanntmachung des Hinweises auf die Patenterteilung : **10.09.86 Patentblatt 86/37**

(84) Benannte Vertragsstaaten : **CH DE FR GB LI NL**

(56) Entgegenhaltungen :
**DE-A- 2 154 370**
**FR-A- 1 538 795**
**GB-A- 1 256 357**
**US-A- 3 840 605**
**JOURNAL OF THE AMERICAN CHEMICAL SOCIETY, band 98, Nr. 25, Dezember 8, 1976, P.J. KROPP et al.: "Photochemistry of alkyl halides. 4. 1-norbornyl, 1-norbornylmethyl, 1- and 2-adamantyl, and 1-octyl bromides and iodides 1", Seiten 8135-8144**
**THE JOURNAL OF ORGANIC CHEMISTRY, Band 47, Heft 10, 7. Mai 1982, American Chemical Society, R.I. DAVIDSON et al.: "Oxidatively assisted nucleophilie substitution/elimination of alkyl iodides in alcoholic media. A further study", Seiten 1904-1909**
**TETRAHEDRON LETTERS, Heft 43, October 1979, Pergamon Press, j.F. BIELLMANN et al.: "Synthèse d'éthers par utilisation d'alcoolate à cation crypté", Seiten 4209-4210**
**TETRAHEDRON LETTERS, Band 30, 1974, Pergamon Press, A. Mc KILLOP et al.: "Mercury-assisted solvolyses of alkyl halides, simple procedures for the preparation of nitrate esters, acetate esters, alcohols and ethers", Seiten 2467-2475**

(73) Patentinhaber : **L. GIVAUDAN & CIE Société Anonyme**
**CH-1214 Vernier-Genève (CH)**

(72) Erfinder : **Chalk, Alan J.**
**5 Duchess Drive**
**Fayson Lakes Kinnelon, N.J. 07405 (US)**
Erfinder : **Purzycki, Kenneth L.**
**273 Marcella Road**
**Lake Parsippany, N.J. 07054 (US)**

(74) Vertreter : **Urech, Peter, Dr. et al**
**Grenzacherstrasse 124 Postfach 3255**
**CH-4002 Basel (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 104 602 B1**

Jouve, 18, rue St-Denis, 75001 Paris, France

CHEMICAL ABSTRACTS, Ninth Collective Index, Band 76-85, 1972-1976, American Chemical Society, Seite 25974CS

Die Akte enthält technische Angaben, die nach dem Eingang der Anmeldung eingereicht wurden und die nicht in dieser Patentschrift enthalten sind.

**Beschreibung**

Die vorliegende Erfindung betrifft Riechstoffkompositionen, die durch einen Gehalt an einem ungesättigten Aether, nämlich der Verbindung 1-(2-Propenoxy)-2,7-octadien (I) gekennzeichnet sind.

Der Aether der Formel I besitzt einen Geruchscharakter, dessen Natur als fruchtig bezeichnet werden kann und demzufolge mit gutem Effekt bei der Herstellung von neuen Riechstofformulierungen insbesondere in Formulierungen für die Verwendung in funktionellen Produkten, wie Seifen, Kosmetika, Detergentien, Reinigungsmittel, etc. verwendet werden können.

Eine Reihe von Methoden zur Herstellung von Alkadienyläthern durch Reaktion eines Diens, wie Isopren oder Butadien, in Anwesenheit eines Alkohols und Katalysators sind bekannt, siehe z. B. US-A-3 499 042 ; GB-A-1 256 357 ; DE-C-1 807 491 ; J. Prakt. Chem. *315* 1067 (1973) ; Chem. Lett. 1329 (1978). Bei der Reaktion von Butadien in Anwesenheit eines Alkyl- oder Alkenylalkohols und eines geeigneten Katalysators bildet sich ein 2,7-Octadien-alkyl- oder -alkenyläther. Wenn anstelle von Butadien Isopren eingesetzt wird, können sich Isomerengemische bilden, deren Natur davon abhängt, ob die Isopreneinheiten Kopf-Schwanz, Schwanz-Schwanz oder Kopf-Kopf addieren. Das überwiegend auftretende Isomere ist der 2,6-Dimethyl-2,7-octadienyl-alkyl oder -alkenyläther, der durch eine Kopf-Schwanz-Dimerisierung entsteht, und der in einer Menge von ungefähr 75 % ± 5 % des Gemisches anfällt. Die in geringeren Mengen anfallenden Isomeren resultieren aus Schwanz-Schwanz-Dimerisierung (3,6-Dimethyl-2,7-octadienyl-alkyl oder -alkenyl-äther) oder der Kopf-Kopf-Dimerisierung (2,7-Dimethyl-2,7-octadien), wobei das erstgenannte Isomere in ungefähr 18 % ± 5 % und das letztere in einer Menge von ungefähr 8 % ± 5 % anfällt. Die Verbindung I ist z. B. aus GB-A-1 256 357 bekanntgeworden.

Die DE-A-2 154 370 beschreibt Aether der Formel

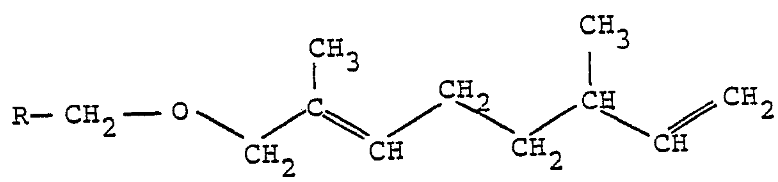

I′

worin R Wasserstoff, geradkettige oder verzweigte, gesättigte oder ungesättigte aliphatische Kohlenwasserstoffreste mit 1 bis 5 C-Atomen oder eine einkernige Arylgruppe darstellt.

Die Verbindungen I′ stellen Riechstoffe dar.

Die FR-A-1 538 795 schliesslich handelt von den Aethern

$$R_1OR_2 \qquad I''$$

worin $R_1$ für Methyl oder Aethyl steht und $R_2$ Alkyl oder Alkenyl mit 5 bis 7 Kohlenstoffatomen darstellt.

Auch diese Verbindungen stellen Riechstoffe dar.

Der Aether der Formel I weist eine sogenannten sauberen (unvermischten, fehlerfreien) und angenehmen fruchtigen Geruchscharakter auf. Der fruchtige Geruch in der Verbindung erinnert an Ananas. Zusätzliche Geruchsnoten, wie grüne Noten, können ebenfalls auftreten.

Die grüne Note steht zusammen mit der fruchtigen Note heraus.

Die saubere Natur der Noten, die auftritt, lässt den Aether der Formel I als besonders geeignet zur Herstellung von Riechstoffkompositionen erscheinen. Bei diesen Noten handelt es sich, wie gesagt, um grüne und fruchtige bzw. Citrusnoten. Der Aether I ist auch nützlich, um Riechstoffkompositionen ganz bestimmte Nuancen zu verleihen, so z. B. kann er den Kopfnoten Intensität verleihen oder die grundlegenden Geruchsnoten verstärken.

Der Aether I, bei dem der grüne und fruchtige Geruch im Vordergrund steht, eignet sich insbesondere bei der Herstellung von Riechstofformulierungen mit krautigem, camphrigen, fichtenartigen, kieferartigen, blumigen Chypre- und Cologne-Charakter, insbesondere in blumigen Basen. 1-(2-Propenoxy)-2,7-octadien ist darum so interessant, weil es bezüglich grünen und fruchtigen Noten ausgesprochen ausgewogen erscheint ; diese Verbindung ist deshalb auch für die Herstellung von Formulierungen dieses Typs geeignet. Die ganz besonders lang andauernde Grünnote als auch der angenehme Charakter dieses Grüns müssen hier hervorgehoben werden. Die Verbindung eignet sich als Komponente von Riechstoffkompositionen dann ausgesprochen, wenn solchen kompositionen eben diese Note verliehen werden soll, oder bei Verwendung in geringen Konzentrationen, beispielsweise ca. 0,2 %, um in diesem Fall spezielle Effekte hervorzurufen.

Das 1-(2-Propenoxy)-2,7-octadien ist geeignet, um blumige Basen, wie grün-blumige Basen und fruchtig-grüne blumige Basen zu verbessern. So kann z. B. eine grün-blumige Formulierung ohne Aether I einen ausgesprochen unfertigen Eindruck erwecken, weil hier die grünen Noten der einzelnen Komponenten feststellbar sind. Auf analoge Weise kann in einer fruchtig-grünen Blumen-Base, die keinen Aether der Formel I enthält, der fruchtige Charakter flach und unentwickelt wirken. Zugabe von 1-(2-Propenoxy)-2,7-octadien zu diesen (oben) genannten Basen verbindet nun die einzelnen grünen Noten in ausgesprochener Weise und entwickelt den fruchtigen Charakter in der fruchtig-grünen Blumen-Komposition. Solche Basen können durch die Zugabe von Verbindungen der Formel I auch bezüglich Kopfnote und erhöhter Intensität in ausgesprochener Weise gewinnen. Die beiden Kompositionen wirken durch Zugabe des genannten Aethers I ausgesprochen ausbalanciert.

In einer bestimmten blumigen Komposition der. Richtung Hyazinthe bewirkt die Zugabe von 1-(2-Propenoxy)-2,7-octadien das Auftreten einer Stengel-grünen Note, die auch in der natürlichen Hyazinthe festgestellt werden kann. Somit wird der grüne Charakter der Riechstoffkomposition durch Zugabe des genannten Aethers I voller und abgerundeter.

Das 1-(2-Propenoxy)-2,7-octadien wird, um in Riechstoffkompositionen bestimmte Nuancen hervorzurufen, vorzugsweise in geringer Konzentration eingesetzt. So kann es z. B. in einer Konzentration von 0,2 % in einer Citrus-Cologne-Base deren Neroli- und Citruscharakter intensivieren und frischer erscheinen lassen.

Die Riechstoffkompositionen, zu welchen der Aether I zugegeben wird, können als Riechstoffbasen zur Herstellung von Parfums, Toilettenwässern und ähnlichem verwendet werden. Dank der Natur des Riechstoffcharakters eignet sich der-Aether I insbesondere bei der Herstellung von Riechstoffbasen, die bei der Herstellung von funktionellen Produkten, wie Handcremes, Coldcremes, Lotionen, Desodorantien, Enthaarungscremes, Shampoos, Seifen, Detergentien, Raumsprays, etc. eingesetzt werden.

Der Aether I kann in Riechstofformulierungen zweckmässigerweise in einem Bereich von ungefähr 0,1 bis ungefähr 15 % eingesetzt werden. Diese Angaben sollen jedoch nicht limitierend verstanden werden, da spezielle Effekte auch noch bei höheren Konzentrationen erzielt werden können, wobei die obere Grenze einzig und allein durch den erwünschten Effekt und die Imagination des Parfumeurs bedingt ist.

Die Riechstoffkompositionen, die den Aether der Formel I als Komponente enthalten, können auf übliche Weise den zu parfümierenden Produkten zugegeben werden, beispielsweise von ungefähr 0,1 % im Falle von Detergentien, bzw. bis zu ungefähr 30 % im Falle von alkoholischen lösungen. Für solche Riechstoffkompositionen, die zur Parfümierung von Reinigungsmitteln verwendet werden, beispielsweise Bleichen, hat sich ein Bereich von ungefähr 0,1 bis ungefähr 0,25 % als praktisch erwiesen. Aber auch in diesen Fällen sollen diese Bereiche nicht limitierend verstanden werden.

## Beispiel 1

1-(2-Propenoxy)-2,7-octadien

Allgemeine Methodik :

1. Ungefähr 2,8 Mole des entsprechenden Diens und 2 Mole des entsprechenden Alkohols werden bei 80-90 °C in einem Druckgefäss in Anwesenheit von 0,096 g Palladiumacetat, 0,11 g Triphenylphosphin und einer 40 %igen Lösung von Triton B (Benzyltrimethylammoniumhydroxid) in 20 ml des reagierenden Alkohols bei autogenem Druck bis zu 18 Stunden umgesetzt.

2. Ungefähr 2 Mol Butadien und 1,5 Mol des entsprechenden Alkohols werden in einem Druckgefäss bei 100 °C in Anwesenheit von 0,088 g Palladiumchlorid, 0,44 g Natrium-p-toluolsulfonat, 4 ml Wasser und einer 40 %igen Lösung von Triton B in 20 ml des reagierenden Alkohols bei autogenem Druck 1 Woche umgesetzt.

Gemäss Verfahren 1 wird auf diese Weise erhalten :

1-(1-Propenoxy)-2,7-octadien aus Butadien und Allylalkohol. Siedepunkt 91-92 °C/10 mmHg ; Geruch : nach Ananas, grün, Opopanax, Lavendel.

## Beispiel 2

A. Grün-blumige Base

| Komponenten | Gewichtsteile |
|---|---|
| Benzylacetat | 65 |
| Benzylisobutyrat | 10 |
| Cyclamenaldehyd | 25 |
| Citronellol | 40 |
| Dimetol® (2,6-Dimethylheptan-2-ol) | 15 |
| Aethyllinalool | 65 |
| Galbanum-öl | 5 |
| Gardenol® (α-Methylbenzylacetat) | 7 |
| Geranylaceton | 120 |
| Ylang-ylang-öl | 20 |
| Hexyl-zimtaldehyd | 80 |
| Isobutyl-acetat | 7 |
| Isobutyl-linalol | 40 |
| Isocyclocitral | 5 |

| | |
|---|---|
| Lilial® (p-tert-Butyl-α-methylhydrozimtaldehyd) | 30 |
| Linalool (synthetisch) | 40 |
| Linalyl-benzoat | 30 |
| Laurine® (Hydroxycitronellal) | 50 |
| Neral | 25 |
| Nerolidol | 60 |
| Phenyl-äthyl-isobutyrat | 13 |
| Phenyl-äthyl-alkohol | 25 |
| Tetrahydrolinalool | 50 |
| Dipropylen-glykol | 153 |
| | 980 |

Die Geruchscharakteristik der obigen grün-blumigen Base wirkt unfertig. Die grünen Noten sind einzeln wahrnehmbar, die Ausgewogenheit fehlt im Gemisch klarerweise. Ein Zusatz von 2 % (20 Teile) von 1-(2-Propenoxy)-2,7-octadien verbindet nun die individuellen grünen Noten der Base und erhöht zugleich den grünen Charakter der Base. Zusätzlich werden Abrundung und Intensität (der Kopfnote) festgestellt, die Komposition wirkt ausbalanciert.

B. Fruchtig-grüne blumige Base

| Komponenten | Gewichtsteile |
|---|---|
| Crysolide® (4-Acetyl-6-tert-butyl-1,1-dimethylindan) | 20 |
| $C_{14}$-Aldehyd | 30 |
| $C_{16}$-Aldehyd (10 % in Dipropylen-glykol) | 2 |
| Isoamylphenyläthyl-äther | 20 |
| Allyl-α-jonon | 50 |
| Citronellal (rein) | 1 |
| Dimethylbenzylcarbinyl-acetat | 30 |
| Dipropylen-glykol | 462 |
| Gardenol® (α-Methylbenzyl-acetat) | 50 |
| Lavandin-öl | 200 |
| Lilial® (p-tert-Butyl-α-methylhydrozimtaldehyd) | 5 |
| 2,4-Dimethyl-3-cyclohexen-1-carboxaldehyd | 5 |
| Verdyl-acetat | 100 |
| | 975 |

Die obige konventionelle Blumenbase wirkt flach und der fruchtige Charakter erscheint nicht genügend entwickelt. Zugabe von 2,5 % (25 Teilen) von 1-(2-Propenoxy)-2,7-octadien verstärkt die fruchtigen Noten der Komposition und verleiht letzterer mehr Diffusion, insbesondere was die Kopfnoten betrifft. Es resultiert eine « fertige », ausgereifte Komposition.

C. Hyzinthe

| Komponenten | Gewichtsteile |
|---|---|
| Benzyl-acetat | 80 |
| Phenyl-äthyl-alkohol | 260 |
| Zimtalkoholsubstitut | 100 |
| Benzyl-acetat | 50 |
| Hexylzimtaldehyd | 20 |
| Indol (rein) | 1 |
| Phenyl-acetaldehyd rein (50 % in Dipropylenglykol) | 10 |
| Heliotropin rekrist. | 20 |
| Fixolide® Prime (7-Acetyl-1,1,3,4,4,6-hexamethyltetralin) | 20 |
| Lilial® (p-tert-Butyl-α-methylhydrozimtaldehyd) | 50 |
| Nelkenblätteröl redist. | 10 |
| Amyl-salicylat | 100 |
| Terpineol Extra | 40 |
| Amylzimtaldehyd | 45 |
| Galbanum (lösl. Harz) | 10 |
| Benzylcinnamat | 50 |
| Phenyl-äthyl-dimethyl-carbinol | 30 |
| Hexenyl-salicylat | 10 |
| Dipropylen-glykol | 54 |
| | 960 |

Die obige konventionelle Hyazinthenkomposition wirkt flach. Es fehlt auch insbesondere die Fülle des natürlichen Charakters der Hyazinthe. Zugabe von 4 % (40 Teile) von 1-(2-Propenoxy)-2,7-octadien resultiert in einer « Stengel »-grünen Note, die der natürlichen Hyazinthenblüte eigen ist ; gleichzeitig wird der grüne Charakter der ganzen Komposition verstärkt. Diese Komposition wirkt nun insbesondere bezüglich Blumennote eindeutig voller und mehr abgerundet.

D. Cologne-Base

| Komponenten | Gewichtsteile |
|---|---|
| Moschusketon | 10 |
| Fixolide® (7-Acetyl-1,1,3,4,4,6-hexamethyltetralin) | 15 |
| Estragol (p-Methoxy-allyl-benzol) | 5 |
| Bergamotte-öl | 180 |
| Dimetol® (2,6-Dimethylheptan-2-ol) | 5 |
| Helional™ (IFF) (2-Methyl-3,4-methylendioxy-hydrozimtaldehyd) | 20 |
| Zitronenöl nicht-sensibilisierend (Furocoumarin-frei) | 130 |
| Lilial® (p-tert-Butyl-α-methylhydrozimtaldehyd) | 20 |
| Linalyl-acetat synth. | 200 |
| Neroli-öl-substitut | 10 |
| Petitgrain-öl rektifiziert | 40 |
| Rosemarin-öl | 40 |
| Terpinyl-acetat | 50 |
| Lavandin rein | 10 |
| Phenyl-äthyl-alkohol | 10 |
| Muskatellersalbei natürl. | 5 |
| Hexyl-zimtaldehyd | 80 |
| Benzyl-acetat | 10 |
| « Oranger »-Kristalle (β-Acetyl-naphthalin) | 5 |
| Benzyl-salicylat | 40 |
| Hen Absolue | 1 |
| Vetiver-acetat B Extra (destill.) | 20 |
| Anaethol | 3 |
| Bay-öl | 3 |
| Dipropylen-glykol | 86 |
| | 998 |

Der obigen Cologne-Base Richtung Citrus fehlt es insbesondere noch an Intensität und Abrundung. Zugabe von 0,2 % (2 Teile) von 1-(2-Propenoxy)-2,7-octadien erhöht den Citrus- und den Neroli-Charakter der Komposition ; es wird Zunahme der Intensität und des frischen Charakters festgestellt.

**Patentansprüche**

1. Riechstoffkomposition, gekennzeichnet durch einen Gehalt an 1-(2-propenoxy)-2,7-octadien.
2. Verwendung von 1-(2-Propenoxy)-2,7-octadien als Riechstoff.

**Claims**

1. An odorant substance composition, characterized by a content of 1-(2-propenoxy)-2,7-octadiene.
2. The use of 1-(2-propenoxy)-2,7-octadiene as an odorant substance.

**Revendications**

1. Composition odorante, caractérisée en ce qu'elle contient du 1-(2-propénoxy)-2,7-octadiène.
2. Utilisation du 1-(2-propénoxy)-2,7-octadiène comme produit odorant.